(19) Europäisches Patentamt
European Patent Office
Office européen des brevets

(11) Publication number: **0 319 012 A2**

(12) **EUROPEAN PATENT APPLICATION**

(21) Application number: 88120114.9

(22) Date of filing: 02.12.88

(51) Int. Cl.4: C07K 17/00 , A61M 1/36 , C12P 21/02 , C07K 13/00 , C12N 15/00 , A61K 35/14

(30) Priority: 04.12.87 US 128931

(43) Date of publication of application: 07.06.89 Bulletin 89/23

(84) Designated Contracting States: AT BE CH DE ES FR GB GR IT LI LU NL SE

(71) Applicant: E.I. DU PONT DE NEMOURS AND COMPANY
Legal Department 1007 Market Street
Wilmington Delaware 19898(US)

(72) Inventor: De Vries, Yuan Lin
5900 Conway Road
Bethesda Maryland 20817(US)
Inventor: Simon, Paul
307 Irving Drive
Wilmington Delaware 19802(US)
Inventor: Robb, Richard John
107 Farm Avenue
Wilmington Delaware 19810(US)
Inventor: White, Charles Thayer
4 Lombardy Drive
Wilmington Delaware 19803(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Immobilized interleukin 2 and interleukin 2 containing a carboxyl-terminal extension.**

(57) Biologically active forms of human IL2 which differ from natural IL2 by the addition of amino acid residues at the carboxyl terminus are prepared by recombinant DNA techniques and can be chemically derivatized without elimination of biological activity. IL2 can be immobilized on a solid support in a biologically active conformation and used for binding of cells bearing IL2 receptors and for generation of lymphokine activated killer (LAK) cells.

EP 0 319 012 A2

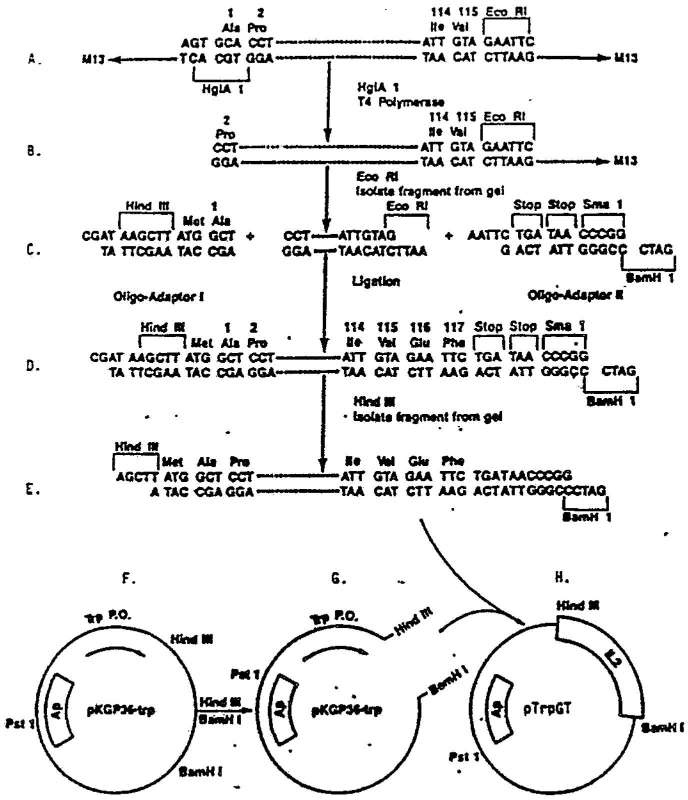

FIG. 1

# IMMOBILIZED INTERLEUKIN 2 AND INTERLEUKIN 2 CONTAINING A CARBOXYL-TERMINAL EXTENSION

## Field of the Invention

The present invention concerns proteins derived by molecular cloning and microbial expression. In particular, the invention relates to the production in E. coli of altered, biologically active forms of human interleukin 2 (IL2) that differ in sequence from natural IL2 by the addition of amino acid residues at the carboxyl terminus of the protein. The invention also relates to biologically functional IL2 immobilized on a solid support.

## Background of the Invention

Interleukin 2 (IL2) is a protein secreted by lymphocytes and belongs to the class of immune modulating substances called lymphokines. IL2 was first described as T-cell growth factor (TCGF), a lymphokine capable of promoting the proliferation of T lymphocytes (Morgan et al. (1976) Science 193, 1007-1008; Ruscetti et al. (1977) J. Immunol. 119, 131-138). IL2 has been shown to modulate many other immunological effects on lymphoid cells including cytotoxic T-cells, natural killer cells, activated B-cells, and lymphokine activated killer (LAK) cells (Robb (1984) Immunol. Today 5, 203 and references therein). IL2 is used to generate LAK cells that kill fresh tumor cells but not normal cells (Grimm et al. (1982) J. Exp. Med. 155, 1823-1841; Mazumder et al. (1984) J. Exp. Med. 159, 495-507). Recently, treatment of cancer patients by administration of IL2 and autologous LAK cells has demonstrated the potential use of IL2 as an immunotherapeutic agent (Rosenberg et al. (1985) N. Eng. J. Med. 313, 1485-1492).

Cellular IL2 receptors (IL2-R) with both high and low affinity for IL2 (Robb et al. (1984) J. Exp. Med. 160, 1126-1146), as well as soluble receptors elaborated by activated lymphocytes (Rubin et al. (1985) J. Immunol. 135, 3172-3177), have been described. The predominant low affinity form of the receptor consists of an approximately 55 kDa protein termed Tac, whereas the high affinity form consists of Tac and one or more additional components (Neeper et al. (1987) J. Immunol. 138, 3532-3538 and references therein). The high affinity form of the IL2-R includes an IL2-binding subunit of 75 kDa molecular weight (Sharo et al. (1986) Science 234, 859-863). Both the TCGF and LAK cell activation signals delivered by IL2 are thought to be mediated by the IL2-R. Soluble IL2 is internalized by responding cells, and apparently this is done by the high affinity IL2-R (Weissman et al. (1986) Proc.Natl. Acad. Sci. 83, 1463-1466; Fujii et al. (1986) J. Exp. Med. 163, 550-562). However, it is not clear from the prior art whether IL2 internalization is necessary for delivering the cellular stimulus for either growth or activation.

It is of interest to examine the biological properties of immobilized IL2 for possible use in, for example, the affinity isolation of lymphocytes bearing the IL2-R and in the production of LAK cells. The conjugation of IL2 and other lymphokines to soluble polymers and insoluble surfaces has been reported in Beecham patent application EP 183503, Asahi patent applications JP 61280432 and JP 61277628, and Kanegafuchi Chem patent application JP 62164470. The Asahi applications, which were published after this invention was made, disclose the generation of LAK cells using immobilized lectin or protein A as primary stimulant and immobilized IL2 as secondary stimulant. Immobilization of IL2 for the purpose of selectively and specifically affinity-precipitating the IL2-R has also been previously reported (Robb and Greene (1983) J. Exp. Med. 158, 1332-1337: Takeda patent JP 61053300). Early attempts to chemically modify IL2 by iodination (Robb (1982) Immunobiol. 161, 21-50) and using other conventional reagents generally led to loss of biological activity, although more recently some successful derivatizations have been reported (Robb et al. (1985) J. Immunol. Meth. 81, 15-30; Hatakeyama et al. (1985) Nature 318, 467-470; Katre et al. (1987) Proc. Nat. Acad. Sci. 84, 1487-1491). Nevertheless, it is clear that IL2 is susceptible to inactivation by chemical modification.

Neeper et al. (1987) J. Immunol. 138, 3532-3538, which was published after this invention was made, discloses coupling IL2 to Sepharose 4B beads and use of the immobilized IL2 to affinity precipitate IL2-R. Neeper et al. describes the IL2 as a specifically engineered form containing a 14 amino acid C-terminal extension including several lysine residues, and having normal in vitro biologic activity and receptor affinity. The C-terminal extended IL2 used by Neeper et al. was the L-IL2 of this invention which is described hereinbelow. Neeper et al. obtained L-IL2 and the description of it from the present inventors. Neeper et al.

does not teach how to make L-IL2 or any utility for it other than affinity precipitation of IL2-R.

Prior to this invention, it was not known whether IL2 could be immobilized on a solid support and still retain its biological activity. It was anticipated that immobilized IL2 might be inactive because, unlike soluble IL2, it could not be internalized by target cells. It was also known that chemical derivatization required for immobilization could alter protein conformation and result in loss of activity.

Molecular cloning of the human IL2 cDNA (Taniguchi et al. (1983) Nature 302, 305-310; Deves et al. (1983) Nucleic Acids Res. 11 4307-4323) has allowed determination of the primary sequence structure of IL2 and the production of large amounts of biologically functional IL2 using heterologous expression systems, including E. coli systems (Ju et al. (1987) J. Biol. Chem. 262 5723-5731 and references therein). Molecular cloning procedures have been used to produce structurally altered forms of IL2 with substitutions and deletions of amino acid residues (Ju et al. op. cit. and references therein). By examining the effects of these structural alterations on the function of IL2, regions of the IL2 protein important for its function have been identified. Biologically functional IL2-derived proteins containing additional amino acid residues at the N-terminus of IL2 have also been reported (Takeda patent EP 1058198: Murphy patent WO 88/0090; Sandoz patent EP 0163603). The production and functional characterization of IL2-derived proteins containing additional amino acid residues at the C-terminus has not been previously reported. Patent applications assigned to Hoechst (DE 3,419,995) and Amgen (WO 85/00817) claim altered forms of IL2 proteins containing additional unspecified amino acid residues at the C-terminus of IL2. However, examples of such altered forms of IL2 are not disclosed. Indeed, previous references show that small deletions or substitutions at the C-terminus of IL2 result in loss of functional activity (Ju et al. op. cit.; Liang et al. (1986) J. Biol. Chem. 261, 334-337).

The present invention provides structurally altered biologically functional forms of IL2 containing additional amino acid residues at the C-terminus. In one embodiment of the invention, IL2 is specifically engineered to contain a 14-amino acid extension at the C-terminus. This 14-amino acid extension facilitates the solubilization, derivatization, and immobilization of the protein in an active conformation.

## Summary of the Invention

The invention provides biologically functional IL2-derived proteins that differ from natural IL2 by the addition of amino acid residues at the C-terminus. These functional altered forms of IL2 are obtained by molecular cloning and expression in E. coli. In one embodiment of the invention, IL2 was engineered to contain a 14-amino acid, lysine-rich extension at the C-terminus. This modified form of IL2 is designated L-IL2. L-IL2 differs from natural human IL2 in that the two C-terminal residues (Leu-Thr) are replaced with:
Gly-Thr-$(Gly)_3$- $(Lys\text{-}Lys\text{-}Asp)_3$-Leu-Glu.

The sequence of the C-terminal extension of IL2 in L-IL2 was selected in order increase its polarity to facilitate solubilization of the protein, and to facilitate the derivatization and immobilization of the protein in a biologically active form. The finding that L-IL2 retains full biological activity indicates that IL2 can be coupled in a biologically active conformation to other functional groups, polypeptide sequences, or solid supports at its C-terminus.

The invention also provides procedures for immobilizing IL2 or derivatives of IL2, such as L-IL2, to a solid support in a conformation that binds to the IL2-R and is active in stimulating T cell proliferation, enhancing natural killer (NK) cell activity and generating LAK cells. Functional, immobilized IL2 is useful in selection and purification of subsets of lymphoid cells bearing IL2-R and as primary, secondary or sole stimulant in generation of LAK cells. Functional, immobilized IL2 can be used in any devices and therapies that involve IL2-mediated biological effects.

## Detailed Description of the Invention

Several derivatives of IL2 have been produced in E. coli using molecular cloning procedures. In human cells, human IL2 is expressed as a precursor protein that is proteolytically cleaved to produce the mature biologically functional form of IL2 (Taniguchi et al., op. cit.; Robb et al. (1983) Proc. Natl. Acad. Sci. 80, 5990-5994). This form of human cell-expressed IL2, designated mature IL2, has an N-terminal Ala residue and is 133 amino acid residues in length. The amino acid residues and corresponding codons of mature IL2 are here designated throughout as amino acid 1 to amino acid 133. For example, amino acid 1 in natural

mature human IL2 is an Ala residue. The E. coli expression vector, and the designation and the structure of the IL2-derived protein encoded by the E. coli expression vector are indicated below in Table 1. The IL2 derivatives expressed in E. coli contain an N-terminal Met initially following translation; however, this Met residue may be subsequently proteolytically removed in E. coli by methionine amino peptidases. N-terminal sequencing of purified r-IL2 has shown that the N-terminal Met is removed in approximately 50% of the molecules purified from E. coli. It is probable that N-Met is removed from approximately the same percentage of other E. coli expressed IL2 derived molecules, including t-IL2, fl-IL2 and L-IL2 (Table 1 and Example 1). The percentage removed can be increased, if desired, by treatment with methionine amino peptidases following isolation and purification.

TABLE 1

| Plasmid | Protein | Protein sequence (1 ... 117 ... 133 ... 148) |
|---|---|---|
| pTrpE-IL2 | r-IL2 | MAP .... FLNRWITFCQSIISTLT |
| pTrpGt | t-IL2 | MAP .... F |
| pTrpGfl | fl-IL2 | MAP .... FLNRWITFCQSIISTGT |
| pTrpGk | L-IL2 | MAP .... FLNRWITFCQSIISTGTGGGKKKDKKDKKDLE |
| Mature, human IL2 | | AP .... FLNRWITFCQSIISTLT (1 ... 133) |

The molecular cloning and E. coli expression procedure are detailed in Example 1 and Example 2. The 14 amino acid C-terminal extension of IL2 in L-IL2, $(Gly)_3$-$(Lys$-$Lys$-$Asp)_3$-Leu-Glu, was specifically designed to facilitate the solubilization and derivatization of the protein. Protein derivatizations often employ the epsilon amino group of lysines. Since covalent attachment of internal lysines to functional groups or solid supports might sterically block subsequent interaction between IL2 and its cellular receptor and thereby interfere with IL2 function, a modified IL2 molecule was designed to contain a lysine-rich hydrophilic segment to provide a chemically accessible derivatization site. The modification was placed at the C-terminus. The Asp residues were interspersed to provide added hydrophilicity and thereby enhance the solubility of the protein in aqueous solution. The Gly residues serve as a spacer between IL2 and the Lys-rich segment of the extension. The Leu-Glu group arises from the nucleotide sequence selected to provide a restriction endonuclease (XhoI) cleavage site.

L-IL2 retains the full biological activities of natural IL2; L-IL2 supports the proliferation of T-cells, augments NK cell activity and induces LAK cell activity. This finding is the first demonstration that amino acid residues can be added to the C-terminus of IL2 without loss of IL2 bioactivity. The E. coli-expressed L-IL2 protein possesses various predicted biochemical properties. Its apparent molecular weight is slightly larger than that of IL2. Its isoelectric point is considerably more basic than IL2 and its hydrophilicity is enhanced.

It is readily possible to derivatize L-IL2 with the amine-reactive biotinylating reagent biotin-SP-NHS (Jackson Immuno Research). The biotin derivatization affords the means to tightly bind IL2 to avidin or streptavidin (Green (1975) Adv. Prot. Chem. 29, 85-133). Biotinylated IL2 is bifunctional in that it is capable of binding the cellular IL2-R and also streptavidin, whether the latter is in solution or attached to a solid surface. Immobilized L-IL2 can immunoprecipitate the Tac IL2-R from cell lysates (Neeper et al., op. cit.). Moreover, it was discovered that streptavidin-mediated immobilized biotinylated L-IL2 retains the ability to bind IL2-R-bearing cells, to induce proliferation and growth of T cells, and to induce LAK cell activity. This is the first demonstration that IL2 can be immobilized and retain its biological activity including the ability to generate LAK cells.

Utilizing standard molecular cloning and expression procedures as described below, IL2 having a C-terminal peptide extension of any desired length, from one to 100 or more amino acid residues, and with any desired amino acid content and sequence, can be produced. Based on the finding that L-IL2 is biologically functional, it is expected that many other such C-terminal extended IL2 derived molecules will also be biologically functional. It is preferred that the extension be at least about 5 amino acid residues in length, and that the amino acids be selected so that the extension will impart to the molecule some desired characteristic, such as reactivity, hydrophilicity, or antigenicity. For derivatization of the molecule, i.e., attachment of functional group or solid support, a C-terminal extension containing at least about 10 mole percent of one or more easily derivatized amino acid residues, such as Lys, Asp, Glu, Met, Ser, Tyr, Thr and Trp, is desirable; Cys can be present, but is less desirable because of potential disulfide bond formation. Preferably, for derivatization, the extension contains at least 2 Lys residues. It is preferred that 2 Lys residues in the extension be adjacent one another and be separated from the carboxyl terminal of the IL2 segment by about 3 or more spacer residues such as Gly. It is most preferred that the extension contain at least 4 Lys residues and that Lys constitute at least 20 mol percent of the extension. For improving the hydrophilicity and solubility of the molecule, an extension containing one or more Asp or Glu residues is desirable. Extension can contain other amino acid residues, including Ala, Val, Leu, Ile, Pro, Phe, Gly, Tyr, Asp, Arg, His and Gln.

The IL2 portion contains substantially the amino acid sequence of human, native IL2. It can be identical in amino acid content and sequence to native, human IL2, or it can be an IL2 mutein such as the fl-IL2 exemplified herein and the muteins described in Ju et al. (1987) J. Biol. Chem. 262 5723-5731 and references cited therein, and in Mark et al., U.S. Patent 4,518,584, the disclosure of which is incorporated herein by reference.

Using procedures of this invention, IL2 can be fused via its C-terminus with polypeptide domains that enhance serum retention, reduce toxicity, or reduce immunogenicity. IL2 can also be fused via its C-terminus to polypeptide domains such as those derived from albumin, transferrin, beta 2 microglobulin, or immunoglobulin recognizing a specific cell marker, to facilitate targeting of the IL2-derived hybrid molecule to specific cells or organs. The increase in molecular size of IL2 by fusion of polypeptide domains at its C-terminus may reduce renal excretion and enhance serum retention time, thereby reducing the effective therapeutic dose and associated toxicity. The C-terminal extension of IL2 can facilitate the chemical derivatization of the molecule in a biologically active state. Derivatization may include the conjugation of IL2 to metal chelating agents for binding of metal radioisotopes or the conjugation of IL2 to photoreactive cross-linking reagents for use in immobilization of IL2 and attachment of IL2 to carrier proteins or cells or particles. IL2 can be fused at its C-terminus to a peptide epitope to facilitate purification and immobilization of the IL2-derived fusion protein using an antibody that recognizes the epitope. Alternatively, a hapten or any other moiety can be chemically derivatized or coupled at the C-terminus of IL2. IL2 can be fused at the C-terminus to a cytotoxic moiety, such as Pseudomonas toxin, and used to selectively kill IL2-R-bearing cells.

The present demonstration that IL2 can be immobilized on a solid support while retaining its ability to generate LAK cells has important applications. Immobilized IL2 is useful for the ex-vivo capture of activated IL2-R-bearing lymphocytes for isolation, further activation, numeric expansion and therapeutic reinfusion in patients with cancer, immunodeficiency diseases, and other diseases. Immobilized IL2 can be used for the on-line adsorption of IL2-receptors found in solution such as in the blood or lymphatic system of patients. This procedure may aid in the reduction of toxic doses of IL2 used in therapy of patients with cancer or immunodeficient conditions. Immobilized IL2 is useful for the on-line activation of blood or lymph cells for improved therapy of cancer, immunodeficiency diseases, and other diseases. Immobilized IL2 may also be used in immunoassays for the detection and quantitation of IL2-receptors, anti-IL2 antibodies and IL2-bearing cells or proteins.

Numerous methods are known for attaching polypeptides containing reactive amino acid residues to solid supports. See Affinity Chromatography and Related Techniques, edited by T.C.J. Gribnau, J. Vissar, R.J.F. Nirard (1982) Elsevier Scientific Publishing, NY, Chapter II, "Polymeric Matrices and Ligand Immobilization," pp. 113-245. Any one of these conventional methods can be used to immobilize the polypeptides of this invention which have a C-terminal extension containing reactive amino acid residues, to provide immobilized polypeptides which have the ability to bind IL2-R, stimulate T cell growth and proliferation, and generate LAK cells. The linkage between the solid support and the IL2 can be covalent and can be direct or indirect, i.e., through a homobifunctional or heterobifunctional cross-linker agent. The covalent linkage can be to the IL2 derived polypeptide, or to an antibody specific for an epitope on the C-terminal extension. A member of an immune or non-immune specific binding pair can be used for immobilization, e.g., an antibody, antigen, hapten or non-immune binding substance can be covalently

attached to the solid support, a specific binding partner of the immobilized member can be covalently attached to the C-terminal extended IL2, and the affinity of the binding pair used to immobilize the IL2 molecule. The preferred method of immobilization is the use of biotin and avidin (or streptavidin) as described above for immobilization of L-IL2. The preferred reagent for biotinylation is biotin-SP-NHS (Jackson Immuno Research) which has the formula:

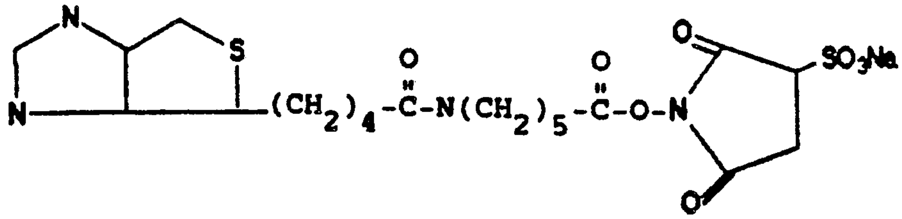

Other biotinylating reagents, such as biotin-NHS (Pierce Chemical Company), which has the formula:

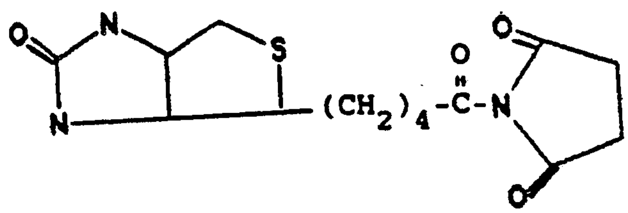

can also be used. The reaction mole ratio of biotinylating agent to IL2 polypeptide with C-terminal extension should be in the range of about 5 to 60.

As illustrated in the examples below, it has also been found that if r-IL2 (no C-terminal extension) is biotinylated using biotin-SP-NHS at a mole ratio of biotin to IL2 in the range of about 5 to 20, and immobilized on an avidin-coated support, the polypeptide retains the ability to bind IL2-R, stimulate proliferation of T cells, and generate LAK cells. This is a surprising finding, because prior attempts to biotinylate IL2 using biotin-NHS at a higher mole ratio resulted in loss of biological activity. This finding indicates that any polypeptide having substantially the amino acid sequence and biological activity of human IL2, regardless of whether it contains a reactive C-terminal extension, can be biotinylated using biotin-SP-NHS and immobilized on an avidin or streptavidin coated support, and the immobilized polypeptide will retain the ability to bind IL2-R and generate LAK cells, provided the reaction mole ratio of biotin to IL2 is maintained in the range of about 5 to 20 for a polypeptide having no C-terminal extension or about 5 to 60 for a polypeptide having a C-terminal extension.

As solid supports there can be used any of the conventional water-insoluble materials used in affinity chromatography and for analyte capture in immunoassays. Preferred materials are organic polymeric beads, films, fibers, plates and tubes. Suitable polymers include polystyrene, nylon, aminoalkyl polyacrylamides, dextran cross-linked with epichlorohydrin, allyl dextran cross-linked with N,N′-methylene bisacrylamide, agarose cross-linked with 2,3-dibromopropanol, cellulose, and many others. Metallic particles, especially magnetic particles, can also be used, such as the particles described in Hersh, U.S. Patent 3,933,997, Forrest et al. U.S. Patent 4,141,687 and Lau U.S. Patent 4,661,408.

E.-coli strain HB101 transformed with plasmid pTrpGk, encoding protein L-IL2 and described in Example 1, has been deposited under the Budapest Treaty with the American Type Culture Collection (ATCC), Rockville, Maryland, and bears Deposit Accession Number 67540. Upon issuance of a patent on this application, all restrictions on the availability of the deposited material to the public will be irrevocably removed.

## Examples

The invention is further described by the following examples, wherein all parts and percentages are by weight and degrees are Celsius.

## Example 1

### Plasmid Constructions

Plasmid constructions were carried out using standard methodology as described by Maniatis et al., Molecular Cloning: A Laboratory Manual, Cold Spring Harbor Laboratory, NY (1982), the teaching of which is hereby incorporated by reference. Enzymes and other reagents used for plasmid constructions were obtained from Bethesda Research Laboratories, Gaithersburg, MD or New England Biolabs, Beverly, MA. Methods for digesting, identifying, recovering, and purifying the various nucleotide sequences used in the invention are known to those skilled in the art as are methods for ligating the sequences into vectors, transforming host microorganism strains, cloning, and recovering products synthesized. Accordingly, the methods will only be described by reference to specific embodiments of the invention set forth hereinafter. Oligonucleotides were synthesized on an Applied Biosystems DNA synthesizer using procedures recommended by the supplier.

### Construction of Plasmid pTrpGt

Plasmid pTrpGt, that expresses in E. coli a C-terminal-truncated IL2, designated t-IL2, was constructed as described below. The IL2 coding sequence was derived from a cDNA library constructed from the gibbon T-cell line MLA-144 as described by Chen et al. (1985) Proc. Natl. Acad. Sci. 82, 7284-7288). A clone from this cDNA library, designated A36, was shown by standard DNA hybridization, restriction endonuclease mapping, and DNA sequence analysis methods to contain a DNA insert corresponding to an IL2 cDNA. Fig. 1A shows A36 in a M13 cloning vector. The N-terminal Ala of mature IL2 (Robb et al. (1983) Proc. Natl. Acad. Sci. 80, 5990-5994) is here designated as amino acid (aa) 1. The mature form of IL2 is 133 aa in length. As shown in Figure 1A, in clone A36 there exists an EcoRI site at the codons corresponding to aa 116 and aa 117.

The IL2 CDNA segment encoding aa 1 to aa 117 was inserted into an E. coli plasmid expression vector using the procedure outlined schematically in Figure 1. Clone A36 DNA was cut with HgiAI, the 3′ single-stranded ends were removed to create blunt ends using T4 DNA polymerase, the DNA was cut with EcoRI, and the blunt-ended HgiAI to EcoRI DNA fragment containing the IL2 coding sequence was isolated (Fig. 1A-1C). The blunt-ended HgiAI end was converted to a HindIII end by ligation to an oligonucleotide designated oligo-adaptor I (Fig. 1C) and the EcoRI end was converted to a BamHI end by ligation to an oligonucleotide designated oligo-adapter I (Fig. 1C). Oligo-adaptor I provides an ATG translation initiation codon 5′ to IL2 codon 1 and oligo-adapter II provides a TGA translation stop codon 3′ to IL2 codon 117. The resultant HindIII to BamHI IL2-containing DNA fragment (Fig. 1D, 1E) was subsequently ligated to the BamHI to HindIII tryptophan (trp) promoter-containing fragment (Fig. 1G) of plasmid, pKPG36-trp (Fig. 1F) (Ivanoff et al. (1986)Proc. Natl. Acad. Sci. 83, 5392-5396). Plasmid expression vector pKGP36-trp provides transcriptional control signals derived from the E. coli tryptophan operon, as well as translation initiation signals. The resultant plasmid, designated pTrpGt, expresses under trp promoter control a C-terminal-deleted IL2-derived protein, designated t-IL2, corresponding to aa 1 to aa 117 of IL2. It should be noted that t-IL2, as with each of the E. coli-expressed IL2-derived proteins described here, differs from authentic IL2 by the addition of an N-terminal Met residue in a portion of the molecules.

### Construction of Plasmid pTrpGfl

In order to facilitate subsequent molecular cloning steps, the HindIII to EcoRI IL2-containing fragment of pTrpGt was inserted between the HindIII and EcoRI sites of the high copy pBR322 derivative, pHC624 (Boros et al. (1984) Gene 30, 257-260), to yield plasmid pHCGt. An IL2 coding sequence closely corresponding to the full length mature IL2 coding sequence was reconstructed by ligating the large IL2-containing BamHI to EcoRI fragment of pHCGt to the following EcoRI to BamHI synthetic oligonucleotide:

```
        117

        Phe Leu Asn Arg Trp Ile Thr Phe Cys Gln

    5'  AA TTC CTG AAC AGA TGG ATT ACC TTT TGT CAA
    3'       G GAC TTG TCT ACC TAA TGG AAA ACA TT

          EcoR1
                                    133
        Ser Ile Ile Ser Thr Gly Thr ***

        AGC ATC ATC TCA ACA GGT ACC TGA TAA CCC GG          3'
        TCG TAG TAG AGT TGT CCA TGG ACT ATT GGG CCC TAG     5'

                                  Kpnl                BamH1
```

The asterisks indicate a translation stop codon. The indicated oligonucleotide extends the IL2 coding sequence from codon 117 to codon 133. The resultant plasmid is designated pHCGfl. The protein product corresponding to the IL2 coding sequence in pHCGfl (fl-IL2) differs from natural human or gibbon ape IL2 only by the substitution of Leu at aa 132 with a Gly residue. Gly was substituted for Leu at aa position 132 as a consequence of the introduction of a KpnI site at this position in the coding sequence. As described below, this KpnI site is used to engineer an extension at the C-terminus of the IL2 protein. The HindIII to BamHI IL2-containing fragment was then ligated to the BamHI to HindIII trp promoter-containing fragment of pKGP36-trp, to yield pTrpGfl. The IL2-derived product encoded and expressed by pTrpGfl is designated fl-IL2.

Construction of Plasmid pTrpGk

The following steps were used to construct plasmid pTrpGk, which encodes an IL2-derived protein differing from natural IL2 by the replacement of the C-terminal Leu-Thr residues by the sequence: Gly-Thr-$Gly_3$-$(Lys\text{-}Lys\text{-}Asp)_3$-Leu-Glu.

This IL2 derivative with a Lys-rich 14 aa C-terminal extension is designated L-IL2.

The KpnI end of the HindIII to KpnI IL2-containing fragment of pTrpGfl was ligated to the KpnI to BamHI oligonucleotide shown below.

```
                    Gly Gly Gly Lys Lys Asp Lys Lys

    5'            C GGA GGA GGT AAG AAG GAT AAG AAG
    3'        CA TGG CCT CCT CCA TTC TTC CTA TTC TTC

       Kpnl

        Asp Lys Lys Asp Leu Glu ***

        GAT AAG AAG GAT CTC GAG TGA TAA CCC GG
        CTA TTC TTC CTA GAG CTC ACT ATT GGG CCC TAG

                                               BamH1
```

The resultant HindIII to BamHI DNA fragment was ligated between the HindIII and BamHI sites of plasmid pHC624, to yield pHCGL. Following confirmation of structure by DNA sequence analysis, the HindIII to BamHI IL2-containing fragment from pHCGL was ligated to the BamHI to HindIII trp promoter-containing fragment of pKGP36-trp, to yield pTrpGk.

Construction of pTrp-IL2

A human IL2 cDNA was obtained from a cDNA library constructed using standard procedures (Gubler and Hoffman (1983) Gene 25, 263-287) from the human Jurkat T-cell line. The double stranded cDNAs were tailed with dCTP and terminal nucleotidyl transferase and then annealed with the G-tailed pBR322. This forms a PstI site at each end of the cDNA. The Jurkat-derived cDNA library was screened by standard hybridization procedures using as probe an oligonucleotide corresponding to a segment of the published human IL2 sequence (Taniguchi et al., op. cit.). One such pBR322-derived plasmid containing a human IL2 cDNA insert was isolated and designated p1H40.

In order to express active mature IL2 at high levels in E. coli, the IL2 cDNA was edited to remove the 5′ DNA coding region for the signal sequence and the edited IL2 cDNA was inserted into an expression vector. Plasmid p1H40 was digested with PstI, and the cDNA insert was purified. An HgiAI restriction site resides at the junction between the last amino acid of the signal sequence and the first amino acid (Ala) of mature human IL2. The PStI fragment containing the IL2 cDNA was cleaved with HgiAI. Since HgiAI leaves a four base pair (bp) 3′ overhang, the Klenow fragment of DNA polymerase was used to remove the four bases and leave a blunt end. This manipulation removed the Ala codon and left a blunt end beginning with CCT, the codon for the second amino acid (Pro) of mature IL2.

In order to supply a translation initiation codon (ATG) as well as the codon for Ala, the following adapter oligonucleotide was synthesized:

```
              Met Ala
CGATAAGCTT    ATG GCT
   TATTCGAA   TAC CGA
     HindIII
```

This oligonucleotide provides an ATG initiation codon, the first codon of mature IL2 (GCT), as well as a HindIII site preceding the ATG. The adapter molecule was ligated to the blunt ended-HgiAI cDNA fragment described above, and the ligated DNA was digested with HindIII and StuI. The HindIII to StuI 450 bp fragment was ligated to the 2325 bp HindIII to PvuII fragment of pBR322 to yield pBREIL-2.

The large IL2-containing HindIII to PStI fragment from pBREIL-2 was ligated to the small trp promoter-containing fragment of pKGP36-trp to yield plasmid pTrpE-IL2. Plasmid pTrpE-IL2 expresses the mature form of human IL2 in E. coli under the transcriptional control of the trp operon promoter. The product of pTrpE-IL2 is designated r-IL2. E. coli-expressed mature IL2 (r-IL2) differs from human cell-expressed mature IL2 by the addition of an N-terminal Met residue. Approximately 50% of mature human IL2 expressed in E. coli has the N-terminal Met residue specifically removed by the action of aminopeptidases in E. coli. The sequence of mature human IL2 expressed in E. coli from pTrpE-IL2 is indicated in Table 1.

## Example 2

### Expression in E. coli and Purification of IL2-Derived Proteins

As described above, E. coli plasmid expression vectors containing the tryptophan promoter were derived from pKGP36-trp (Ivanoff et al., op. cit.). E. coli host strains MM294 and HB101, were used (Maniatis et al., Molecular Cloning: A Laboratory Manual, Cold Spring Harbor Laboratory, NY (1982)). Expression from the tryptophan promoter was carried out as described (Ivanoff et al.,op. cit.).

Each of the IL2-derived proteins, t-IL2, fl-IL2, and L-IL2, accumulated to high level in E. coli, representing approximately 5 to 10% of total E. coli protein. Greater than 95% of the IL2-derived protein was expressed as an aggregate that remained insoluble under conditions that solubilize most E. coli proteins. As a result, the insoluble, aggregated IL2-derived protein can be highly purified by rapid and simple extraction and centrifugation steps. Many products are known to aggregate in a relatively insoluble form following high level expression in E. coli (see Marston (1986) Biochem. J. 240, 1-12).

Following induction of IL2-derived protein expression in E. coli, the cells were collected by centrifugation, suspended in buffer TE (10 mM Tris HCl, pH 7.6, 0.1 mM EDTA), and again pelleted by centrifugation and suspended in buffer TE. The E.< coli cells were lysed by sonication (three 10 second pulses) and the cell lysate was centrifuged (27,000 x g, 30 minutes). Following centrifugation, the pellet of sonicated bacteria was extracted with 50% acetic acid or 80% acetic acid plus 10% methanol. After stirring for 30 min at ambient temperature, the suspension was centrifuged at 10,000 RPM (SS34 rotor, Sorvall) for 10 min. The supernatant was diluted 3-fold with 0.1% trifluoroacetic acid (TFA) in water and filtered through a 0.8 micron filter (Corning). The filtrate was loaded directly on 1 x 25 cm Ultrapore RPSC reverse-phase column (Beckman). The column was developed with a linear gradient of increasing acetonitrile concentration in 0.1% TFA. The L-IL2 eluted at 40-45% acetonitrile, in contrast to the normal elution of E. coli-expressed human IL2, which elutes at 52-56% acetonitrile. The material in the HPLC peak was concentrated 2-fold under a stream of nitrogen. On SDS-polyacrylamide gel electrophoresis L-IL2 appeared to be pure. Glucose was added to a concentration of 0.3 M and the solution was dialyzed for 10 hours against 0.3 M glucose. The L-IL2 was activated by heating at 65°C for 2 hours followed by rapid cooling in an ice bath. The solution was filtered through a 0.22 micron filter.

## Example 3

## TCGF Activity of E. coli-expressed IL2-derived Proteins

The TCGF bioactivity of IL2 was measured using the murine cytolytic T-cell line CTLL-2 (Gillis and Smith (1977) Nature 268, 154-156). These cells grow only in the presence of IL2, and their growth is directly proportional to the IL2 activity in the cultures. The overnight growth of CTLL cells in the presence of varying amounts of IL2 can be measured by either the uptake of [methyl-$^3$H]thymidine (Gillis et al. (1978) J. Immunol. 120, 2027-2032) or by the cells' ability to metabolize the dye MTT (Mosman (1983)J. Immunol. Meth. 65, 55). Typically, 20,000 CTLL-2 cells are plated in 96-well microtiter plates in medium either lacking IL2 or containing serially diluted concentrations of a standardized IL2 preparation. One unit of TCGF activity was defined as the reciprocal of the dilution that yielded half-maximal incorporation of [methyl-$^3$H]thymidine or optical density change of MTT. Unknown IL2 preparations are measured against a standard curve of standard IL2 on a logit plot (Robb (1982) Immunobiol. 161, 21-50). Table 2 shows the IL2 TCGF bioactivity of crude and HPLC-purified E. coli-expressed r-IL2, t-IL2, fl-IL2, and L-IL2.

TABLE 2

TCGF Activity of IL2-Derived Proteins

| Sample | Crude *E. coli* Lysates (units/ml) | Purified Protein (units/mg) |
|---|---|---|
| r-IL2 (pTrpE-IL2) | 30,873 | 110,526 |
| t-IL2 (pTrpGt) | 0 | ND |
| fl-IL2 (pTrpGfl) | 43,890 | ND |
| L-IL2 (pTrpGk) | 23,366 | 122,222 |
| Negative Control (pTrp36-trp) | 0 | ND |

Induced *E. coli* containing the plasmid indicated in parentheses were lysed by sonication in buffer TE and the cell lysate was centrifuged. The crude *E. coli* lysate supernatant was diluted in TE containing 0.01% SDS prior to TCGF assay. IL2-derived proteins were also assayed following HPLC purification. TCGF activity was determined in the CTLL assay by logit analysis using purified Jurkat (mammalian) IL2 as the standard.

As shown in Table 2, the TCFG activity of fl-IL2 and L-IL2 is essentially the same as that for r-IL2. These results show that substitution of Leu 132 with Gly, as in fl-IL2, or the 14 aa C-terminal extension in L-IL2 do not alter the TCFG activity of the molecule. In contrast, deletion of 15 C-terminal aa residues from IL2, as in t-IL2, results in total loss of TCGF activity. Thus, although the C-terminal residues are critical for TCGF activity, it is here demonstrated that the addition of aa residues to the C-terminus of IL2 does not impair TCGF activity.

Example 4

IL2-Dependent Enhancement of Natural Killer Cell Activity in Human Peripheral Blood Lymphocytes

Circulating normal lymphocytes exhibit the ability to kill certain cultured tumor cell lines (Herberman and Oraldo (1981) Science 214, 24). This ability is markedly enhanced following overnight incubation with IL2 (Trinchieri et al. (1984) J. Exp. Med. 160, 1147-1169).

Non-adherent human peripheral blood lymphocytes were obtained after Ficoll-Hypaque centrifugation and plastic adherence, using procedures described (Boyum, A. (1968) Scand. J. Clin. Lab. Invest. 21 - (Suppl. 97): 77-89). These lymphocytes were used as effector cells in a short-term (4 hour) $^{51}$[Cr] release assay against K562 erythroleukemia cells (ATCC #CCl-243) which had been labeled with sodium $^{51}$[Cr], using procedures described (Muul et al. (1986) J. Immuno. Methods 88, 265).

The effector cells were incubated for approximately 18 hours in the presence of different IL2 preparations. Table 3 shows the results of these experiments indicating that L-IL2 possesses the ability to enhance NK activity. E. coli containing the appropriate plasmid were induced for expression of the indicated protein. Following induction, E. coli cells were lysed by sonication and the cell lysate was centrifuged (27,000 x g, 30 minutes). The resultant supernatant was diluted as indicated in Table 3 in buffer TE containing 0.01% SDS.

TABLE 3

Generation of NK Cell Activity by IL2-Derived Proteins

| Activator(1) | Dilution Factor | Effector to Target Cell Ratio(2) | | | |
|---|---|---|---|---|---|
| | | 60 | 20 | 7 | 2 |
| No activator | - | 28 | 10 | 3 | 1 |
| fl-IL2 | 15,000 | 78 | 50 | 13 | 4 |
| | 45,000 | 80 | 49 | 16 | 4 |
| | 135,000 | 73 | 42 | 12 | 4 |
| | 405,000 | 67 | 36 | 10 | 4 |
| L-IL2 | 15,000 | 80 | 46 | 13 | 5 |
| | 45,000 | 70 | 29 | 9 | 6 |
| | 135,000 | 58 | 26 | 7 | 7 |
| | 405,000 | 50 | 24 | 9 | 5 |
| r-IL2 | 15,000 | 85 | 60 | 20 | 7 |
| | 45,000 | 76 | 61 | 19 | 8 |
| | 135,000 | 79 | 49 | 15 | 6 |
| | 405,000 | 65 | 37 | 13 | 8 |
| E. coli Extract | 15,000 | 60 | 30 | 12 | 7 |

(1) IL2 TCGF bioactivities of samples prior to dilution (measured in the CTLL bioassay) were 9,100 units/ml (fl-IL2), 17,000 units/ml (L-IL2) and 14,500 units/ml (r-IL2). Sonicated bacterial lysates were diluted in buffer TE contai ing 0.01% SDS.
(2) $^{51}$[Cr]-labeled K562 cells were used as targets; data are presented as percent of $^{51}$[Cr] released.

## Example 5

### Generation of Lymphokine-Activated Killer (LAK) Cell Activity in Human Peripheral Blood Lymphocytes

Human peripheral blood lymphocytes were obtained by Ficoll-Hypaque centrifugation (Boyum, op. cit.) and incubated in the presence of 10 TCGF units/ml of r-IL2, L-IL2 or B-L-IL2 (see Example 6 below), for 3 to 4 days. LAK cell cytotoxicity of the cultured cells was measured using $^{51}$[Cr]-labeled Daudi cells (ATCC #CCL-213) as target in a 4-hour assay. L-IL2 and B-L-IL2, just as native IL2, are capable of inducing LAK activity in human peripheral blood lymphocytes following exposure of these cells to IL2 for 3 days (Table 4). Similar results were obtained using Raji cells (ATCC #CCl-86) as targets (not shown).

TABLE 4

| Generation of LAK Cell Activity by IL2-Derived Proteins | | | | | |
|---|---|---|---|---|---|
| Lymphokine | Ratio of Effector Cell to Target Cell(1) | | | | |
| | 50 | 20 | 8 | 3 | 1 |
| r-IL2(2) | 56 | 29 | 17 | 5 | 2 |
| L-IL2(2) | 52 | 50 | 25 | 12 | 5 |
| B-L-IL2 | 56 | 33 | 13 | 5 | 0 |

(1) Target cell is $^{51}$[Cr]-labeled Daudi cells; data is presented as percent of total $^{51}$[Cr] released.
(2) 10 unit/ml E. coli-expressed IL2 purified by HPLC as described in text.

## Example 6

### Biotinylation of L-IL2 and r-IL2

Purified L-IL2 and r-IL2 were derivatized with the biotinylating reagent biotin-SP-NHS (Jackson ImmunoResearch). Reactions were conducted in 0.1 M $NaHCO_3$. The biotin reagent was dissolved in dimethyl formamide (DMF) and added to the aqueous L-IL2 solution to a final DMF concentration not to exceed 10%. The mixture was agitated for 1 hour at ambient temperature (approximately 25° C). Residual biotin-SP-NHS reactivity was quenched by the addition of 1/5 volume of 0.1 M glycine or ethanolamine, and the mixture was agitated for a further 30 min. The reaction was dialyzed against saline containing 0.3 M glucose using membranes with a 6000 dalton exclusion at 4° C. L-IL2 biotinylated in this manner is designated B-L-IL2; r-IL2 biotinylated in the manner is designated B-IL2.

B-L-IL2 and B-IL2 retain biological activity in the TCGF CTLL-2 bioassay (Table 5). Biotinylation was carried out at differing molar ratios of biotin to L-IL2 or r-IL2. As shown in Table 5, biological activity is reduced by excessive biotinylation and optimal bioactivity is found at biotin to IL2 molar ratios of 5 to 20. As shown in Table 5, L-IL2 retains greater TCGF activity than r-IL2 following reaction with biotin-SP-NHS. This result is consistent with the preferential reaction of biotin-SP-NHS with Lys residues in the C-terminal extension of L-IL2.

TABLE 5

| TCGF Activity if Biotinylated L-IL2 and r-IL2 | | | | | |
|---|---|---|---|---|---|
| | Molar Ratio of Biotin-SP-NHS to IL2 | | | | |
| | 0 | 5 | 10 | 20 | 40 |
| B-IL2 | 10 | 4.7 | 6.3 | 6.2 | 5.0 |
| B-L-IL2 | ND | 8.1 | 8.4 | 9.7 | 5.6 |

(1) TCGF activity (units/ml) of B-L-IL2 and B-IL2 was measured in the CTLL bioassay. Aliquots (50 µg) of purified r-IL2 and L-IL2 were reacted with biotin-SP-NHS at the indicated molar ratios. Reaction products were titered agains a 10 units/ml unreacted r-IL2 standard by logit analysis. ND = not done.

B-L-IL2 was shown to retain essentially full ability to generate LAK cell activity. The specific activity of B-L-IL2 in this assay was similar to that of L-IL2 and r-IL2. See Table 4, Example 5.

B-L-IL2 and B-IL2 were shown to bind to IL2-R on HUT-102 cells. Following incubation with B-L-IL2 or B-IL2 at 4°C for 30 minutes and washing, the HUT-102 cells were incubated further for 30 minutes in the presence of streptavidin (StAV) bearing fluorescein (StAV-FITC) (Sigma) and analyzed in a Spectrum III flow cytometer. Cells became strongly fluorescent only in the presence of B-L-IL2 or B-IL2 (data not shown), indicating the bifunctionality of the biotinylated IL2 (Table 6). In addition, this binding was specific for the cellular IL2-R, since it could be inhibited by preincubation of the cells for 30 minutes with excess IL2 (100 units/ml) or anti-Tac antibody (ascites diluted 1:2000). B-L-IL2 generated under low biotin to L-IL2 molar ratios exhibited better specificity, i.e., ability of binding to be competed by IL2 or anti-Tac antibody.

TABLE 6

Simultaneous Binding of B-L-IL2 to Streptavidin-FITC and IL2-R on HUT-102 Cells[1]

| | Molar Ratio of biotin-SP-NHS to IL2 | | | | | |
|---|---|---|---|---|---|---|
| | 0 | 10 | 20 | 40 | 80 | 160 |
| B-L-IL2 | | | | | | |
| No competition | 18 | 70 | 75 | 58 | 60 | 20 |
| IL2 competition | 15 | 31 | 41 | 27 | 24 | 19 |
| anti-Tac competition | 17 | 23 | 30 | 26 | 22 | 17 |

(1) Data are presented as percent of fluorescent cells, i.e., percent of cells labeled with FITC.

These results demonstrate the bifunctionality of B-L-IL2. B-L-IL2 can simultaneously bind cellular IL2-R on HUT-102 cells and streptavidin (StAv) bearing fluorescein (StAv-FITC). Similar specific bifunctional binding to other IL2-R bearing cells (e.g., PHA blasts, CTLL-2) has also been observed (data not shown).

As shown in Table 7, B-L-IL2 appears to bind more efficiently to IL2-R-bearing HUT-102 cells than does B-IL2. This result indicates that the Lys-rich C-terminal tail present in L-IL2 facilitates the derivatization of IL2 in an active conformation. B-IL2 and B-L-IL2 were generated by the reaction of either IL2 or L-IL2, respectively, with biotin-SP-NHS, at biotin:IL2 mole ratio of 5, then compared for ability to bind simultaneously streptavidin-FITC and IL2-R on HUT-102 cells. B-IL2 and B-L-IL2 were incubated at various concentrations with 200,000 HUT cells and 20 µg/ml streptavidin-FITC in 96-well dishes. The percent of fluorescent cells was determined by flow cytometry, as described above. Results are shown in Table 7.

TABLE 7

Simultaneous Binding of B-IL2 and B-L-IL2 to Streptavidin-FITC and IL2-R on HUT-102 Cells(1)]

| | -log Concentration µg/ml | | | | | |
|---|---|---|---|---|---|---|
| | 1 | 1.5 | 2 | 2.5 | 3 | 3.5 |
| B-IL2 | 98 | 94 | 86 | 38 | 24 | 7 |
| B-L-IL2 | 99 | 98 | 97 | 87 | 68 | 15 |

(1) Data are presented as percent fluorescent cells.
(2) Preincubation of cells for 30 minutes with either IL2 (100 units/ml) or anti Tac antibody (ascites diluted 1:2000) prior to adding B-L-IL2 at 0.1 µg/ml reduced the % fluorescent cells to about 10.

Example 7

Biological Activity of immobilized E. coli-Expressed IL2-Derived Proteins

Earlier attempts by the present inventors to immobilize bioactive IL2 were unsuccessful. Effort to do this by passive adsorption to polystyrene or anchoring with anti-IL2 antibody resulted in reversible attachment. Chemical derivatization of IL2 generally caused the loss of biological activity.

Polystyrene culture dish surfaces were covalently coated with StAv using the carbodiimide coupling reagent 1-ethyl-3-(3-dimethylaminopropyl)carbodiimide-HCl (CDI). The bottom surface of the dishes (petri dishes, 24- or 96-well culture plates (Costar)) was activated by treatment with 1 mg/ml CDI in 0.1 M sodium acetate buffer, pH 4.8 or 0.2 M MES buffer (Sigma), pH 5.5 for 30 min at 37° C. After rinsing with acetate buffer, StAv (Sigma) was added at different concentrations (see Table 8) for 16 h. Unbound StAv was washed repeatedly and residual reactivity of the plastic was quenched by adding glycine or ethanolamine (0.1 M, 30 min, 37° C), followed by 1 mg/ml bovine serum albumin (BSA) (2 to 16 h, ambient room temperature). The vessel was washed 3 times with Hank's balanced salt solution (HBSS) containing 2% fetal calf serum (FCS). B-L-IL2 or B-IL2 (see Example 6) was added at 0.1 $\mu g/mm^2$ and allowed to bind at 4° C for 30 minutes. The wells were washed with 0.2 M sodium citrate buffer, pH 2.5, containing 1 M NaCl. This step was quickly repeated 3 times and followed by 10 washes with HBSS/FCS.

CTLL-2 cells were plated in 96-well microtiter dishes covalently coated with StAv and loaded with B-L-IL2 or B-IL2. The overnight IL2 bioactivity was measured in the CTLL-2 assay and compared with a standard titration series using soluble IL2. The results are shown in Table 8.

TABLE 8

TCGF Activity of Immobilized B-IL2 AND B-L-IL2(1)

| | $\mu g$ StAv/mm² | | | |
|---|---|---|---|---|
| | 0.1 | 0.33 | 1 | 3.3 |
| Im. B-IL2 | 0.007 | 0.079 | 0.098 | 0.074 |
| Im. B-L-IL2 | 0.045 | 0.069 | 0.067 | 0.076 |

(1) Data are presented as "equivalent units/ml", i.e., the concentration of soluble IL2 which provides equal activity.

As shown in Table 8, immobilized IL2 of this invention generated low but measurable TCGF activity. No unbound IL2 was detected in the wells following the coating process (data not shown). This finding is in contrast with the proposed mechanism of IL2-mediated signal transduction requiring receptor-ligand internalization following binding. B-L-IL2 was slightly more effective as an immobilized stimulator of CTLL-2 growth than B-IL2, once more suggesting less destructive biotinylation and/or better presentation on the coated surface.

Immobilized IL2 of this invention also functions in the generation of LAK cells, as shown in Table 9. Human peripheral blood lymphocytes isolated by Ficoll-Hypaque fractionation were cultured for 3 days at 37° C in 24 well dishes coated as described above with immobilized B-IL2 or B-L-IL2 prepared as described for Table 7, or in wells containing soluble r-IL2 at 10 TCGF units/ml. Cytolytic activity was measured against $^{51}$[Cr]-labeled Daudi target cells at various effector cell to target cell ratios. Lymphokine-activated-killer (LAK) activity was detectable in this system using the immobilized IL2 as shown in Table 9.

TABLE 9

| Generation of LAK Cells by Immobilized B-IL2 and B-L-IL2(1) | | | | |
|---|---|---|---|---|
| | Effector:Target Ratio | | | |
| | 40 | 20 | 20 | 2 |
| Soluble IL2 | 70 | 57 | 37 | 21 |
| Im. B-IL2 | 35 | 20 | 12 | 7 |
| Im. B-L-IL2 | 23 | 21 | 13 | 5 |

(1) Data are presented as % $^{51}$[Cr] release.

It appears that IL2 need not be internalized following binding to the IL2-R on the cell surface in order to stimulate T-cell proliferation or mediate the production of LAK cells.

## Claims

1. Polypeptide having substantially the amino acid sequence of human IL2, immobilized on a solid support, and having the ability to bind an IL2 receptor, to stimulate proliferation or growth of T cells, and generate LAK cells.

2. Composition of Claim 1 wherein the polypeptide consists essentially of a segment having substantially the amino acid sequence of human IL2 and a carboxyl-terminal fused peptide extension.

3. Composition of Claim 2 wherein the fused peptide extension contains lysine.

4. Composition of Claim 1 wherein a lysine of the polypeptide is attached to biotin, the support is attached to avidin or streptavidin, and the polypeptide is immobilized on the support through binding of biotin to avidin or streptavidin.

5. Composition of Claim 3 wherein a lysine of the polypeptide is attached to biotin, the support is attached to avidin or streptavidin, and the polypeptide is immobilized on the support through binding of biotin to avidin or streptavidin.

6. Composition of Claim 3 wherein the carboxyl-terminal fused peptide extension contains at least 2 lysine residues and the peptide is at least 10 mole percent lysine.

7. Composition of Claim 5 wherein the carboxyl-terminal fused peptide extension contains at least 2 lysine residues and the peptide is at least 10 mole percent lysine.

8. Composition of Claim 6 wherein at least two lysine residues in the extension are adjacent.

9. Composition of Claim 7 wherein at least two lysine residues in the extension are adjacent.

10. Composition of Claim 8 wherein the extension contains at least 4 lysine residues and the peptide is at least 20 mole percent lysine.

11. Composition of Claim 9 wherein the extension contains at least 4 lysine residues and the peptide is at least 20 mole percent lysine.

12. Composition of Claim 10 wherein the IL2 segment of the polypeptide corresponds to human IL2 except that leucine at position 132 is replaced by glycine, and wherein the carboxyl-terminal fused peptide extension is GGGKKDKKDKKDLE.

13. Composition of Claim 11 wherein the IL2 segment of the polypeptide corresponds to human IL2 except that leucine at position 132 is replaced by glycine, and wherein the carboxyl-terminal fused peptide extension is GGGKKDKKDKKDLE.

14. Composition of Claim 2 wherein the fused peptide extension contains an epitope, the support is attached to an antibody to the epitope, and the polypeptide is immobilized on the support through binding of the antibody to the epitope.

15. Method of capturing human cells having IL2 receptors which comprises contacting the cells with a composition of Claim 1.

16. Method of augmenting NK activity or generating LAK cells which comprises contacting human lymphocytes with a composition of Claim 1.

17. Method of stimulating proliferation or growth of T cells which comprises contacting human T lymphocytes with a composition of Claim 1.

18. Method of treatment of a patient with cancer or immunodeficient condition which comprises contacting ex vivo blood, blood fraction or lymph from the patient with a composition of Claim 1 to capture active IL2-R bearing lymphocytes, further activating and numerically expanding the cells by contact with an activating amount of a lymphokine, and reinfusing the cells into the patient.

19. in the method of treatment of a patient with cancer or immunodeficient condition by administration of IL2, the improvement which comprises contacting ex vivo blood or lymph from the patient with a composition of Claim 1 to remove soluble IL2-R therefrom and returning the blood or lymph to the patient.

20. Method of Claim 16 wherein the lymphocytes are contacted with the immobilized polypeptide on-line and returned to the patient.

21. Polypeptide consisting essentially of a segment having substantially the amino acid sequence of human IL2 and a carboxyl-terminal fused peptide extension, and having the ability to bind an IL2 receptor, stimulate proliferation or growth of T cells, and generate LAK cells.

22. Polypeptide of Claim 21 wherein the fused peptide extension contains aspartic and/or glutamic acid.

23. Polypeptide of Claim 21 wherein the peptide extension contains lysine.

24. Polypeptide of Claim 23 wherein the fused peptide tail contains aspartic and/or glutamic acid.

25. Polypeptide of Claim 24 wherein the peptide extension contains at least 2 lysine residues and the peptide extension is at least 10 mole percent lysine.

26. Polypeptide of Claim 25 wherein at least two lysine residues in the extension are adjacent.

27. Polypeptide of Claim 26 wherein the extension contains at least 4 lysine residues and the extension is at least 20 mole percent lysine.

28. Polypeptide of Claim 27 which corresponds to human IL2, except that leucine at position 132 is replaced by glycine, and wherein the extension is GGGKKDKKDKKDLE.

29. Polypeptide of Claim 21 conjugated to a molecule selected from carrier proteins, antibodies, toxins, haptens, hormones, enzymes, drugs, component of a non-immune binding pair, metal chelating agent, photoactive/photoreactive compounds.

30. Polypeptide of Claim 23 conjugated through lysine to biotin, the reaction mole ratio of biotin to polypeptide being in the range of about 5 to 60.

31. Biotinylated human IL2 having substantially the biological activity of native human IL2, the mole ratio of biotin to polypeptide being in the range of about 5 to 20.

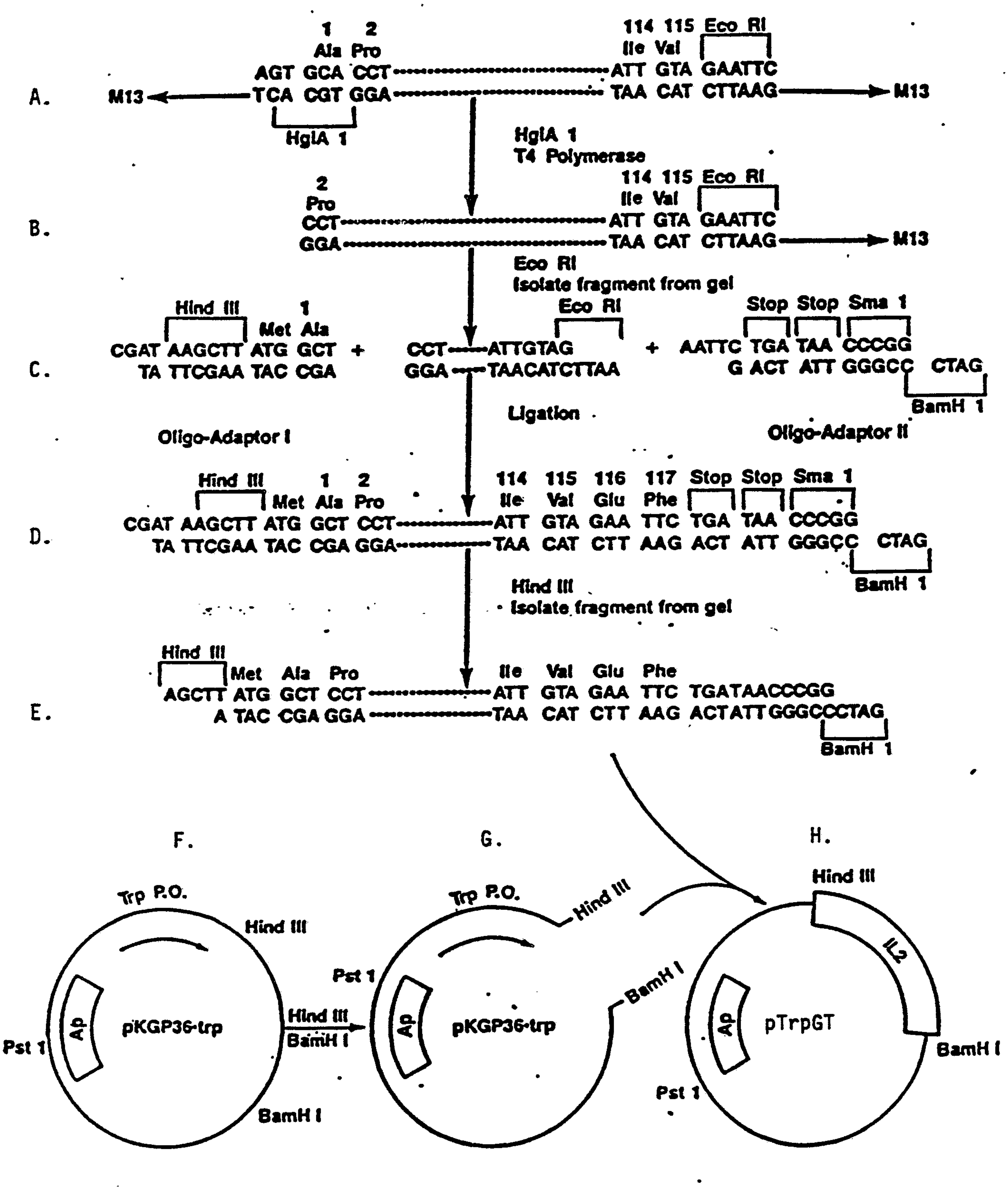

A.
1 Ala 2 Pro
AGT GCA CCT
TCA CGT GGA
M13
HglA 1
114 115 Eco RI
Ile Val
ATT GTA GAATTC
TAA CAT CTTAAG
M13
HglA 1
T4 Polymerase
B.
2 Pro
CCT
GGA
114 115 Eco RI
Ile Val
ATT GTA GAATTC
TAA CAT CTTAAG
M13
Eco RI
Isolate fragment from gel
C.
Hind III 1 Met Ala
CGAT AAGCTT ATG GCT +
TA TTCGAA TAC CGA
Oligo-Adaptor I
Eco RI
CCT ATTGTAG
GGA TAACATCTTAA
+
Stop Stop Sma 1
AATTC TGA TAA CCCGG
G ACT ATT GGGCC CTAG
BamH 1
Oligo-Adaptor II
Ligation
D.
Hind III Met 1 Ala 2 Pro
CGAT AAGCTT ATG GCT CCT
TA TTCGAA TAC CGA GGA
114 Ile 115 Val 116 Glu 117 Phe Stop Stop Sma 1
ATT GTA GAA TTC TGA TAA CCCGG
TAA CAT CTT AAG ACT ATT GGGCC CTAG
BamH 1
Hind III
Isolate fragment from gel
E.
Hind III Met Ala Pro
AGCTT ATG GCT CCT
A TAC CGA GGA
Ile Val Glu Phe
ATT GTA GAA TTC TGATAACCCGG
TAA CAT CTT AAG ACTATTGGGCCCTAG
BamH 1
F.
Trp P.O.
Hind III
Ap
pKGP36-trp
Pst 1
BamH I
Hind III
BamH I
G.
Trp P.O.
Hind III
Pst 1
Ap
pKGP36-trp
BamH I
H.
Hind III
IL2
Ap
pTrpGT
BamH I
Pst 1


FIG. 1